Europäisches Patentamt

European Patent Office

Office européen des brevets

(19)

(11) EP 0 664 449 B1

## (12) FASCICULE DE BREVET EUROPEEN

(45) Date de publication et mention
de la délivrance du brevet:
**14.06.2000 Bulletin 2000/24**

(51) Int Cl.[7]: **G01N 33/00**

(21) Numéro de dépôt: **95400002.2**

(22) Date de dépôt: **02.01.1995**

(54) **Procédé et dispositif de fourniture de gaz à un analyseur de traces d'impuretés dans un gaz**

Verfahren und Vorrichtung zur Lieferung von Gas an eine Vorrichtung zur Analyse von Verunreinigungsspuren in einem Gas

Process and apparatus for feeding gas to a gas impurity trace analyser

(84) Etats contractants désignés:
**BE DE FR IT NL**

(30) Priorité: **07.01.1994 FR 9400120**

(43) Date de publication de la demande:
**26.07.1995 Bulletin 1995/30**

(73) Titulaire: **L'AIR LIQUIDE, SOCIETE ANONYME POUR
L'ETUDE ET L'EXPLOITATION DES PROCEDES
GEORGES CLAUDE
75321 Paris Cédex 07 (FR)**

(72) Inventeurs:
 • **Ronge, Catherine
 F-75013 Paris (FR)**
 • **Mail, Alain
 F-91210 Draveil (FR)**
 • **Marot, Yves
 F-78530 Buc (FR)**

(74) Mandataire: **Vesin, Jacques et al
L'AIR LIQUIDE, S.A.,
Service Propriété Industrielle,
75, Quai d'Orsay
75321 Paris Cédex 07 (FR)**

(56) Documents cités:
EP-A- 0 479 633          EP-A- 0 519 783
EP-A- 0 528 386          FR-A- 2 219 373
US-A- 3 776 023

**Description**

La présente invention est relative à un procédé et à un dispositif de fourniture de gaz à un analyseur de traces d'impuretés dans un gaz et, plus particulièrement, à un tel procédé et un tel dispositif suivant lesquels on fournit séquentiellement à l'analyseur un gaz à analyser, un gaz pur et un gaz d'étalonnage obtenu par dilution d'une ou plusieurs impuretés dans ce gaz pur.

On connaît, du document FR-A-2 667 397 au nom de la demanderesse, un tel procédé et un tel dispositif conçus pour l'alimentation d'un analyseur à très haute sensibilité. Un tel analyseur peut être conçu pour détecter des impuretés à de très faibles concentrations ($10^{-2}$-$10^{-5}$ ppm par exemple). Il doit alors être souvent étalonné par fourniture d'un gaz pur ou "zéro" et de gaz "d'étalonnage" contenant des impuretés à des concentrations précisément déterminées.

Dans le dispositif du document précité, on assure un débit de gaz constant dans chacune des lignes fournissant à l'analyseur le gaz à analyser, un gaz pur et un gaz d'étalonnage chargé d'une quantité prédéterminée d'impuretés, respectivement, en disposant dans chaque ligne un orifice calibré fonctionnant en régime sonique et un capteur de pression en amont de cet orifice, la pression captée commandant un régulateur de débit placé dans une dérivation de décharge de la ligne, à l'aval de l'orifice. Pour éviter que le capteur de pression ne puisse polluer le gaz circulant dans la ligne, le capteur est monté sur une dérivation équipée d'une ligne de fuite autorisant un faible débit de fuite dans cette dérivation. Toutes ces précautions accroissent l'encombrement du dispositif ainsi que sa complexité et son coût de fabrication. L'encombrement du dispositif est encore grevé par l'utilisation d'une bouteille remplie de gaz d'étalonnage. Par ailleurs, la précision du réglage du débit est affectée par celle de la mesure de pression opérée par le capteur, qui doit elle-même être corrigée en température quand celle-ci varie.

La présente invention a donc pour but de fournir un procédé et un dispositif du type décrit ci-dessus, conçus de manière à autoriser une réalisation plus compacte du dispositif tout en assurant un réglable plus précis des débits de gaz à fournir à l'analyseur.

La présente invention a aussi pour but de fournir un tel procédé et un tel dispositif, garantis contre toute pollution des gaz fournis par des organes tels que capteurs de pression, régulateurs de pression ou de débit, et permettant de changer rapidement et commodément de gaz d'étalonnage de l'analyseur.

On atteint ces buts de l'invention, ainsi que d'autres qui apparaîtront à la lecture de la description qui va suivre, avec un procédé de fourniture de gaz à un analyseur de traces d'impuretés dans un gaz, suivant lequel on fournit séquentiellement à l'analyseur a) un gaz à analyser, b) un gaz pur et c) un gaz d'étalonnage obtenu par dilution d'une ou plusieurs impuretés dans ce gaz pur, ce procédé étant remarquable en ce qu'on alimente un ensemble d'au moins deux lignes dérivées montées en parallèle avec ce gaz pur, on charge le gaz pur dérivé d'une des lignes avec une quantité prédéterminée d'au moins une impureté pour obtenir, après dilution avec le gaz pur dérivé de l'autre ligne dérivée, le gaz d'étalonnage, qui est le long d'une ligne d'alimentation dirigée vers l'analyseur et on divise le débit de gaz pur admis dans l'ensemble à l'aide de deux restrictions calibrées placées chacune à l'entrée des lignes dérivées. Le débit de gaz pur alimentant l'ensemble est régulé à l'aide d'un régulateur de débit placé en amont de l'ensemble.

Comme on le verra plus loin, l'utilisation de restrictions calibrées dans des lignes raccordées en parallèle, en combinaison avec une régulation du débit alimentant la dérivation permet de constituer des moyens de division de débit compacts et précis, sans mesure de pression ou de température, et sans qu'il soit nécessaire de mettre en oeuvre des étapes et moyens de calcul, traditionnellement associés à ces mesures.

La restriction calibrée pourra consister en tout moyen permettant de réaliser cette division, qu'il s'agisse, par exemple, d'un orifice, d'un capillaire, ou encore d'un fritté.

Pour la mise en oeuvre du procédé selon l'invention, on utilise un dispositif comprenant a) une source de gaz pur b) un ensemble d'au moins deux lignes de gaz dérivées montées en parallèle et alimentées par la source de gaz pur, c) des moyens pour charger le gaz pur dérivé circulant dans une des deux lignes avec une quantité prédéterminée d'au moins une impureté, d) une restriction placée à l'entrée de chacune des lignes dérivées et calibrée pour diviser dans un rapport prédéterminé, entre ces deux lignes, le débit de gaz pur alimentant l'ensemble et e) un régulateur du débit de gaz pur alimentant l'ensemble, situé entre la source de gaz pur et l'entrée de gaz de l'ensemble, et pour chaque ligne dérivée, la portion de ligne entre l'entrée de gaz de l'ensemble et la restriction calibrée est dépourvue de tout organe de mesure de pression.

Suivant une caractéristique du dispositif selon l'invention, celui-ci comprend en outre deux régulateurs de débit placés chacun dans une ligne de décharge connectée, pour l'une, à la sortie de la ligne dérivée de gaz chargée d'impuretés et, pour l'autre, à la sortie de l'ensemble.

Cette disposition permet d'assurer un réglage précis des débits de gaz dans les deux lignes et du taux de dilution du gaz d'étalonnage chargé d'impuretés délivré par une de ces lignes, dans le gaz pur délivré par l'autre ligne. Avantageusement, le dispositif comprend des moyens permettant d'établir au moins un deuxième étage de dilution du gaz d'étalonnage.

Selon l'une des mises en oeuvre de l'invention, le dispositif comprend en outre un épurateur de gaz placé entre la source de gaz pur et l'entrée de gaz de l'ensemble.

Suivant une autre caractéristique avantageuse du dispositif selon l'invention, les moyens pour charger d'impuretés le courant de gaz pur dérivé circulant dans une des lignes dérivées en parallèle pour constituer le gaz d'étalonnage, sont constitués par une pluralité de cartouches de perméation débitant sélectivement ou collectivement, en parallèle, des impuretés dans ladite ligne, avec des taux de perméation prédéterminés. Ces cartouches de perméation, particulièrement compactes, permettent de réduire l'encombrement du dispositif. L'utilisation d'un jeu complet de telles cartouches permet de réaliser commodément et rapidement un gaz d'étalonnage de telle ou telle composition prédéterminée.

Comme il apparaîtra clairement à l'homme du métier, le procédé selon l'invention permet d'effectuer l'étalonnage de l'analyseur en lui fournissant selon les cas (type d'analyseur, choix de l'utilisateur), séquentiellement, du gaz pur et du gaz d'étalonnage pollué en impureté(s), ou encore, par exemple, séquentiellement, deux mélanges différents d'étalonnage comprenant des teneurs différentes en impureté(s).

D'autres caractéristiques et avantages de la présente invention apparaîtront à la lecture de la description qui va suivre et à l'examen du dessin annexé dans lequel :

-    la figure 1 représente schématiquement la circuiterie et divers organes d'un premier mode de réalisation du dispositif selon l'invention,
-    la figure 2 représente schématiquement une variante d'une partie du dispositif de la figure 1, et
-    la figure 3 est un graphe représentant l'évolution de la concentration en impuretés d'un gaz d'étalonnage, lors de la formation de celui-ci à l'aide du dispositif selon l'invention.

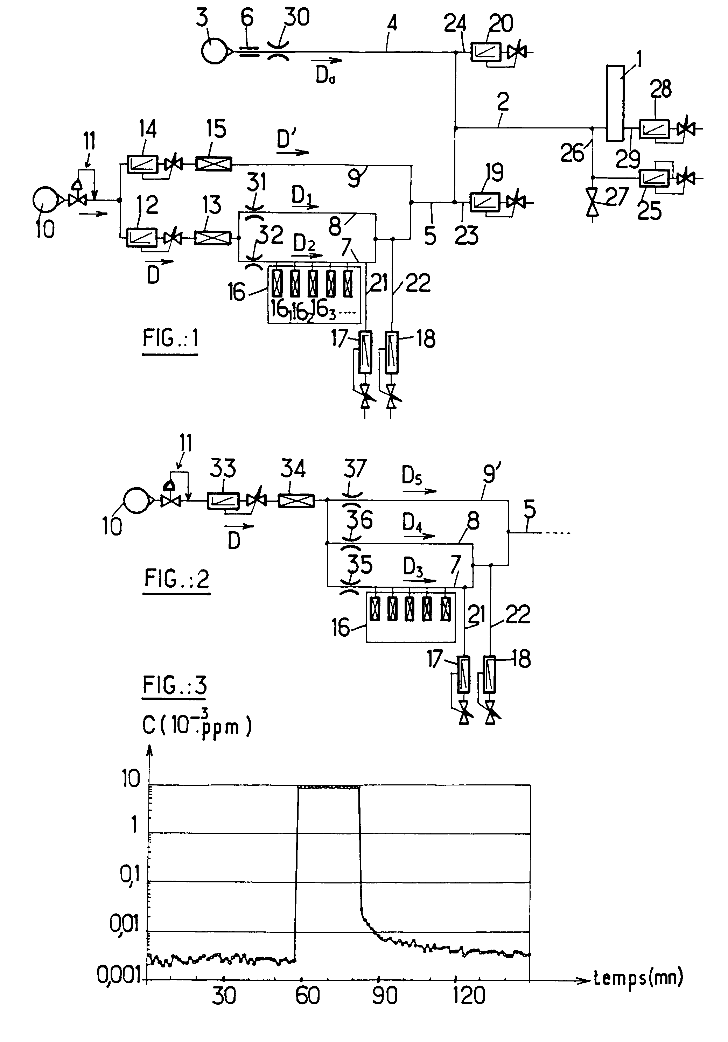

On se réfère à la figure 1 où l'on a représenté un premier mode de réalisation du dispositif suivant l'invention, conçu pour la fourniture de gaz à un analyseur 1 à très haute sensibilité, capable de mesurer des traces d'impuretés présentes à des concentrations du domaine $10^{-2}$-$10^{-5}$ ppm par exemple. C'est ainsi que ce dispositif doit permettre d'assurer 1) la génération d'un gaz pur de référence ou "gaz zéro" c'est-à-dire en fait d'un gaz épuré contenant moins de $10^{-5}$ ppm d'impuretés, 2) la génération d'un gaz d'étalonnage contenant des traces de gaz ou impuretés, par exemple $H_2O$, $CO_2$, $CO$, $O_2$, $CH_4$, $H_2$, etc... à des concentrations variables dans une plage s'étendant de $10^{-5}$ ppm à $10^{-2}$ ppm, et 3) le contrôle de paramètres d'introduction du gaz dans l'analyseur, tels que la pression et le débit.

Pour ce faire, le dispositif représenté à la figure 1 comprend une ligne d'alimentation 2 de l'analyseur 1 qui peut être raccordée sélectivement à une source 3 d'un gaz à analyser par l'intermédiaire d'une ligne de raccordement 4, ou à une ligne 5 débitant soit un gaz d'étalonnage, soit un gaz pur, pour les besoins de l'étalonnage de l'analyseur, comme cela est bien connu. Cette ligne 5 est elle-même alimentée par des première, deuxième et troisième lignes 7,8,9 respectivement, alimentées par une deuxième source de gaz 10, qui peut d'ailleurs être constituée par la source 3 elle-même. La source 10 est raccordée aux trois lignes 7,8,9 à travers un régulateur de pression 11, la sortie du régulateur de pression étant connectée d'une part à l'ensemble des lignes dérivées 7,8 qui sont montées en parallèle, à travers un régulateur de débit 12 et un épurateur 13 et, d'autre part, à la ligne additionnelle 9 à travers un régulateur de débit 14 et un épurateur 15. Des moyens 16, qui seront décrits en plus de détail dans la suite, sont prévus pour charger le courant de gaz traversant la ligne 7 avec des impuretés constituées par des traces de gaz tels que $CO$, $CH_4$, $O_2$, $CO_2$, $H_2O$, par exemple.

Des régulateurs de débit 17,18,19,20 sont montés sur des lignes de décharge 21,22,23,24 respectivement, de la ligne 7, de la sortie des lignes 7 et 8, de la ligne 5 et de la ligne 4, respectivement.

Le contrôle des paramètres d'introduction du gaz dans l'analyseur 1 est assuré à l'aide d'un régulateur de pression amont 25 monté en dérivation sur une ligne de décharge 26 de la ligne 2, cette ligne de décharge comprenant en outre une vanne de purge 27, et à l'aide d'un régulateur de débit 28 monté sur la sortie 29 de gaz de l'analyseur.

Enfin, le débit de gaz dans la ligne 4 peut être ajusté et mesuré, classiquement, à l'aide d'un orifice calibré 30 en amont duquel est placé un capteur de pression non polluant, tel qu'un capteur piézo-électrique 6 en ligne, du type "through tube" par exemple, suivant sa dénomination anglo-saxonne.

Il est essentiel que les débits de gaz $D_1$,$D_2$ dans les lignes 8 et 7 respectivement soient fixés avec précision, puisque de ces débits dépend le taux de dilution des impuretés injectées par les moyens 16, à la sortie de ces lignes. Suivant une caractéristique essentielle de la présente invention, on atteint ce résultat à l'aide de restrictions calibrées, pour le mode de réalisation représenté de simples orifices calibrés 31,32 montés à l'entrée des lignes dérivées 7,8 respectivement et du régulateur de débit 12 (le cas échéant à l'aide de régulateurs additionnels 17 et 18) qui leur est associé.

On privilégiera pour les orifices calibrés du système un fonctionnement en régime sonique, mais le procédé et dispositif selon l'invention ont montré des résultats satisfaisants et comparables même en s'éloignant quelque peu du régime particulier que constitue le régime sonique.

En écoulement sonique du gaz à travers les orifices 31 et 32, c'est-à-dire quand pour chaque orifice le rapport de la pression amont à la pression aval est supérieur à 2, on sait que les débits massiques $D_1$ et $D_2$ du gaz dans ces orifices 32,31 respectivement sont égal à :

$$D_1 = K_{GAZ}.P.T^{-1/2}.S_1 = \alpha.P.S_1$$

$$D_2 = K_{GAZ}.P.T^{-1/2}.S_2 = \alpha.P.S_2$$

avec

$D = D_1 + D_2$, débit total du gaz fourni aux lignes 7 et 8,

$P$ = pression en amont des orifices

$T$ = température, commune aux deux orifices si ceux-ci sont proches,

$K_{GAZ}$ = constante ne dépendant que de la nature du gaz et de la géométrie de l'orifice, ces géométries étant supposées identiques (par exemple circulaires) pour les deux orifices 31 et 32,

$S_1, S_2$ = sections des orifices 31,32 respectivement.

Des relations précédentes on tire :

$$D_1 = D.S_1/(S_1 + S_2)$$

$$D_2 = D.S_2/(S_1 + S_2)$$

On remarquera que, pour un débit total D donné, la précision $\Delta D_i/D_i$ sur chacun des débits ne dépend de la précision atteinte sur le rapport des sections $S_1$ et $S_2$ soit, pour $D_1$ :

$$\Delta D_1/D_1 = \Delta(S_2/S_1)/(1 + S_2/S_1)$$

La pression amont P des orifices s'équilibre automatiquement à la valeur :

$$P = D/(\alpha.(S_1 + S_2))$$

Pour que, dans le domaine des débits D considéré, les orifices 31 et 32 soient utilisés en régime sonique, la somme des sections $S_1 + S_2$ de ces orifices doit être telle que pour chaque orifice on ait :

$$P/P_{AVAL} > 2$$

ou $P_{AVAL}$ est la pression en aval de l'orifice considéré.

La division de débit ainsi obtenue n'exige ainsi, avantageusement, ni mesure de pression ni mesure de température, au bénéfice de la compacité, de la simplicité et du coût de fabrication du dispositif suivant l'invention. Le principe de division ainsi établi peut être généralisé à un ensemble de N orifices calibrés comme on le verra plus loin en liaison avec la figure 2.

Pour des conditions s'éloignant légèrement du régime sonique (rapport de pression égal à 2 ou un peu moins de 2), le débit arrivant sur l'ensemble se reparti néanmoins dans le rapport des surfaces des orifices calibrés (ou encore dans le rapport des pertes de charge introduites par un capillaire ou un fritté).

Néanmoins, plus les conditions s'éloigneront d'un tel rapport 2 et plus le système sera sensible à des variations de pression aval.

En ce qui concerne la régulation et le contrôle des débits dans les diverses lignes du dispositif suivant l'invention, on remarquera que tous les composants susceptibles de polluer le gaz, au niveau de $10^{-5}$ ppm ou plus par exemple, tels qu'une vanne ou un régulateur de débit, ont été placés systématiquement soit en amont des épurateurs (régulateurs de débit 12,14, régulateurs de pression 11) soit en dérivation ou en aval des lignes critiques (régulateurs de débit 17,18,19,20,25).

On revient aux moyens 16 utilisés pour charger le gaz circulant dans la ligne 7 avec des traces de gaz ou impuretés, et pour diluer le gaz ainsi chargé de manière à obtenir des gaz "étalons" de composition précisément déterminée. Suivant l'invention, ces moyens sont avantageusement constitués par une batterie de cartouches de perméation ($16_1,16_2,16_3$, etc...) chargées de gaz tels que $O_2,CO_2,H_2O,CH_4,CO,H_2$, etc... Ces cartouches peuvent être montées en parallèle sur la ligne 7, pour débiter en permanence et simultanément dans celle-ci, ou bien encore pour être séparément branchées sur la ligne 7 à travers de simples vannes à deux ou trois voies, qui peuvent être considérées comme non polluantes aux fortes concentrations en impuretés du gaz de la ligne 7, avant dilution dans le gaz apporté par les lignes 8 et 9. Ces cartouches sont couramment de faible encombrement (2x2x10 cm par exemple) et peuvent donc restées montées à demeure dans le dispositif, sous un faible volume. En choisissant des cartouches dont le taux de perméation $\tau$ est faible, la durée de vie d'une telle cartouche peut être alors supérieure à 1 an.

La concentration C du gaz de débit $D_2$ s'écoulant dans la ligne 7, en impuretés introduites par une cartouche $16_i$ de taux de perméation $\tau$ est de :

$$C_0 = K\tau/D_2$$

$\tau$ ne dépendant de la nature d'une membrane de perméation équipant la cartouche, du gaz et de la température, K étant la constante du gaz considéré.

On comprend que l'introduction d'une batterie 16 de cartouches de perméation $16_i$ dans le dispositif suivant l'invention assure à celui-ci une grande compacité, une longue durée de vie et offre un grand choix dans les gaz que l'on peut introduire pour constituer les gaz d'étalonnage de l'analyseur. Ces gaz d'étalonnage sont obtenus après dilution du gaz sortant de la ligne 7 dans le gaz pur de débit $D_1$ sortant de la ligne 8, puis la dilution du gaz résultant dans le gaz pur de débit D' sortant de la ligne 9, dilutions contrôlées par les régulateurs de débit 17,18.

Suivant une caractéristique avantageuse du dispositif selon l'invention, toutes les lignes du dispositif sont continuellement balayées par du gaz, de façon à maintenir les surfaces intérieures des lignes en équilibre dynamique d'absorption/désorption, pour éviter des régimes transitoires en pression/débit pendant lesquels toute surface en contact avec le gaz est susceptible de désorber et d'adsorber des molécules, processus susceptible de modifier la composition des gaz formés.

Suivant encore une autre caractéristique avantageuse du dispositif selon l'invention, toutes les sorties des régulateurs de débit massiques ainsi que celles de la ligne 27 de purge sont réunies et collectées en une sortie unique (non représentée) dirigée vers un épurateur par exemple, avant rejet ou recyclage éventuel. On empêche ainsi toute modification de la composition des gaz formés, par rétrodiffusion possible de gaz à travers ces sorties. En outre, il est possible alors d'utiliser des gaz inflammables ou toxiques qui demandent des précautions particulières.

On va maintenant expliquer le fonctionnement du dispositif selon l'invention, d'une part en phase d'analyse d'un gaz de débit $D_a$ provenant de la source 3, d'autre part en phase d'étalonnage de l'analyseur à l'aide d'un gaz "zéro" et de gaz d'étalonnage.

En phase d'analyse, il faut évidemment que les lignes 21,22 et 5 absorbent entièrement les gaz débités par les lignes 7,8 et 9 pour que ceux-ci n'entrent pas dans la ligne 2 d'alimentation de l'analyseur. Les débits $D_{ij}$ fixés par les régulateurs ij concernés doivent alors satisfaire à la relation :

$$D_{19} + D_{17} + D_{18} > D_{12} + D_{14}$$

Soit

$$(D_{19} + D_{17} + D_{18}) - (D_{12} + D_{14}) = R > 0$$

Le débit R excédentaire doit alors provenir de la ligne 4.

Si on fixe aussi à R le débit de fuite dans la ligne 24 (pour y maintenir une circulation de gaz), le débit de gaz $D_{28}$ dans l'analyseur satisfait à la relation :

$$D_{28} < D_a - 2R$$

L'écart à l'égalité provenant du débit de fuite dans le régulateur de pression 25 et de pertes éventuelles dans l'analyseur.

Dans un mode de réalisation particulier du dispositif suivant l'invention, où les débits $D_1, D_2$ dans les lignes 8 et 7 respectivement sont égaux et établis à l'aide d'orifices soniques 31,32 circulaires calibrés à 62 µm, où les débits maximum des régulateurs 12,14,17,18,19,20 et 24 sont respectivement de 190 cc/mn, 5 1/mn, 100 cc/mn, 7 1/mn, 10 1/mn et 5 1/mn, on a choisit R = 200 cc/mn.

Pour l'envoi d'un gaz d'étalonnage pur ou "zéro" dans l'analyseur 1, il faut court-circuiter le débit $D_a$ de la ligne 4 dans la ligne de fuite 24 et celui de la ligne 7 dans la ligne 21. Avec les paramètres de réglage des débits indiqués ci-dessus, on peut choisir alors :

$$D_{14} = 5 \text{ l/mn}$$

$$D_{12} = 190 \text{ cc/mn} = D$$

$$D_{17} = D_{18} = 100 \text{ cc/mn}$$

$$D_{19} = R$$

$$D_{20} = D_A + R$$

$$R = 200 \text{ cc/mn par exemple}$$

Pour l'envoi dans l'analyseur 1 du seul gaz d'étalonnage formé dans la ligne 7, il faut court-circuiter le débit $D_a$ comme indiqué ci-dessus et régler la dilution des impuretés dans le gaz fourni par la source 10 par un réglage approprié des débits des lignes 21 et 22, grâce au régulateur de débit 17 et 18 respectivement soit, par exemple :

$$D_{14} = 5 \text{ l/mn}$$

$$D_{12} = 190 \text{ cc/mn} = D$$

$$D_{19} = R$$

$$D_{17} < D_2$$

$$D_{18} < D - D_{17}$$

$$D_{20} = D_A + R$$

La concentration C du gaz ou mélange étalon fourni à l'analyseur est de la forme :

$$C = C_0 \times Q_1 \times Q_2$$

où $C_0 = K\tau/D_2$ comme on l'a vu plus haut et $Q_1, Q_2$ sont les facteurs de dilution des premier et deuxième étages de dilution, respectivement, que l'on peut ajuster à l'aide des régulateurs de débit 17 et 18 respectivement, le produit $Q_1 \times Q_2$ pouvant alors prendre toute valeur comprise entre $5.10^{-5}$ et $2.10^{-2}$ par exemple.

Avec un gaz pur constitué par de l'oxygène et une cartouche de perméation de taux de perméation $\tau$ = 50 ng/mn, on a pu ainsi former des mélanges étalons à des teneurs comprises entre $1,8.10^{-5}$ ppm et $7,5.10^{-3}$ ppm.

Il apparaît ainsi que la dilution des impuretés dans le gaz d'étalonnage et la commutation des trois types de gaz à fournir à l'analyseur peuvent être obtenues par la seule commande des divers régulateurs de débit du dispositif selon l'invention. Celui-ci se prête donc à une automatisation dans laquelle le réglage de ces régulateurs serait assuré, par exemple, par un calculateur dûment programmé à cet effet.

On a représenté à la figure 2 une variante (7,8,9') de l'agencement des lignes 7,8,9 de la figure 1 et des régulateurs et épurateurs associés. Dans cet agencement, on utilise un seul régulateur de débit 33 et un seul épurateur 34 pour alimenter les trois lignes, la ligne 9' supplémentaire étant raccordée entre l'entrée commune des lignes 7 et 8 et la sortie commune de ces lignes. Les débits $D_3$, $D_4, D_5$ dans les lignes 7,8,9' sont alors fixés par des orifices soniques calibrés 35,36,37 respectivement, de sections $S_3, S_4, S_5$ respectivement, de diamètre $d_3, d_4, d_5$, suivant les relations:

$$D_3 = D \times S_3/(S_3 + S_4 + S_5)$$

$$D_4 = D \times S_4/(S_3 + S_4 + S_5)$$

$$D_5 = D \times S_5/(S_3 + S_4 + S_5)$$

Avec $d_3 = d_4 = 60\,\mu m$ et $d_5 = 400\,\mu m$ par exemple, on retrouve les performances du dispositif de la figure 1 avec :

$$D_3 = D_4 = 95 \text{ cc/mn}$$

$$D_5 = 5000 \text{ cc/mn}$$

en bénéficiant d'un encombrement, d'une complexité et d'un coût de fabrication moindres du dispositif, du fait de la suppression d'un régulateur et d'un épurateur.

Le dispositif suivant l'invention peut être associé, par exemple, à un appareil d'analyse constitué par un spectromètre de masse à ionisation à la pression atmosphérique, pour lui fournir, entre autres, un gaz zéro contenant moins de $2.10^{-6}$ ppm d'oxygène et moins de $10^{-5}$ ppm d'eau. La figure 3 donne le graphe fourni par un tel spectromètre lors de la commutation de son alimentation entre un gaz zéro et un mélange à 0,009 ppm d'oxygène, le graphe montrant que cette commutation s'opère sensiblement instantanément.

Il apparaît maintenant que l'invention permet d'atteindre les objectifs fixés, à savoir réaliser un dispositif de fourniture de gaz à un analyseur qui soit, à la fois, précis, de conception simple et d'encombrement réduit, notamment du fait de la suppression de toute prise de pression en amont des restrictions, et autres étapes de calcul qui leur étaient associées, à mise en oeuvre et purge rapides, présentant un temps de réponse très court lors d'un changement de concentration d'impuretés pendant l'étalonnage, permettant de changer simplement et rapidement de gaz d'étalonnage grâce à la batterie de cartouches de perméation utilisée, et qui soit d'automatisation aisée.

Bien entendu l'invention n'est pas limitée aux modes de réalisation décrits et représentés, qui n'ont été donné qu'à titre d'exemple. Ainsi, on ne sortirait pas du cadre de la présente invention en multipliant le nombre de lignes montées entre la source 10 et la ligne 5, pour accroître le nombre des étages de dilution du gaz d'étalonnage au-delà des deux étages observés sur les figures 1 et 2.

## Revendications

1. Procédé de fourniture de gaz à un analyseur (1) de traces d'impuretés dans un gaz, suivant lequel on fournit séquentiellement à l'analyseur a) un gaz à analyser, b) un gaz pur et c) un gaz d'étalonnage obtenu par dilution d'une ou plusieurs impuretés dans ce gaz pur, caractérisé en ce que ledit gaz d'étalonnage est fourni à l'analyseur de la façon suivante :

   (i) on alimente, avec le gaz pur, un ensemble d'au moins deux lignes (7, 8) dérivées, disposées en parallèle, le débit de gaz pur dérivé circulant dans chaque ligne dérivée étant obtenu en divisant le débit de gaz pur qui alimente l'ensemble à l'aide de restrictions calibrées (31, 32) placées chacune à l'entrée de chacune des lignes dérivées,

   (ii) on charge le gaz pur dérivé, d'une (7) des lignes dérivées, avec une quantité prédéterminée d'au moins une impureté, pour obtenir, après dilution avec le gaz pur dérivé de l'autre ligne dérivée, le gaz d'étalonnage, qui est, le long d'une ligne d'alimentation, dirigé vers l'analyseur,

   (iii) on régule le débit de gaz pur alimentant l'ensemble à l'aide d'un régulateur de débit placé en amont (12) de l'ensemble.

2. Procédé selon la revendication 1, caractérisé en ce qu'on régule le débit de gaz d'étalonnage obtenu à la sortie de gaz de l'ensemble avec un régulateur de débit (17) placé dans une ligne de décharge (21) connectée à la sortie de la ligne dérivée de gaz chargé d'impuretés en amont de la sortie de gaz de l'ensemble et un autre régulateur de débit (18) placé dans une ligne de décharge (22) connectée à la sortie de gaz de l'ensemble.

3. Procédé selon la revendication 1 ou 2, caractérisé en ce que le gaz d'étalonnage subit, en sortie d'ensemble, avant d'atteindre l'analyseur, une autre étape de dilution, par l'apport d'une quantité additionnelle de gaz pur (9, 9', 14, 15).

4. Procédé selon l'une quelconque des revendications 1 à 3, caractérisé en ce que lesdites restrictions calibrées sont des orifices calibrés.

5. Procédé selon la revendication 4, caractérisé en ce que lesdits orifices calibrés sont utilisés en régime sonique.

6. Dispositif pour la mise en oeuvre du procédé selon l'une quelconque des revendications 1 à 5, comprenant :

   a) une source de gaz pur (10);
   b) un ensemble d'au moins deux lignes (7, 8) de gaz dérivées, montées en parallèle et alimentées par la source (10) de gaz pur, la sortie de chaque ligne dérivée étant raccordée à une sortie de gaz commune constituant la sortie de gaz de l'ensemble, qui est connectée à une ligne (2) d'alimentation de l'analyseur (1) ;
   c) des moyens (16) pour charger le gaz pur dérivé circulant dans une des lignes dérivées, avec une quantité prédéterminée d'au moins une impureté ;
   d) une restriction placée à l'entrée de chacune des lignes dérivées, calibrée pour diviser entre les lignes dérivées dans un rapport prédéterminé, le débit de gaz pur alimentant l'ensemble ;
   e) un régulateur du débit de gaz pur alimentant l'ensemble, situé entre la source de gaz pur et l'entrée de gaz de l'ensemble,

   pour chaque ligne dérivée, la portion de ligne entre l'entrée de gaz de l'ensemble et la restriction calibrée étant dépourvue de tout organe de mesure de pression.

7. Dispositif selon la revendication 6, caractérisé en ce qu'il comprend en outre, deux régulateurs de débit (17, 18) placés chacun dans une ligne de décharge (21, 22) connectée, pour l'une, à la sortie de la ligne (7) dérivée de gaz chargée d'impuretés, en amont de la sortie de gaz de l'ensemble, et, pour l'autre, à la sortie de gaz de l'ensemble.

8. Dispositif selon la revendication 6 ou 7, caractérisé en ce qu'il comprend un épurateur, situé entre la source de gaz pur et l'entrée de gaz de l'ensemble.

9. Dispositif selon l'une quelconque des revendications 6 à 8, caractérisé en ce qu'il comprend en outre une ligne (9) additionnelle, alimentée en gaz pur par la source (10) à travers un régulateur de débit (14) et un épurateur (15), la sortie de cette ligne (9) étant raccordée, de même que la sortie de gaz de l'ensemble à la ligne (2) d'alimentation de l'analyseur (1).

10. Dispositif selon la revendication 6 ou 7, caractérisé en ce qu'il comprend en outre une troisième ligne de gaz (9'), les trois lignes étant alimentées par la source (10) de gaz pur à travers un même régulateur de débit (33) et un même épurateur (34), l'entrée de la troisième ligne (9) étant munie d'une restriction calibrée (37), assurant, avec les deux autres (35, 36), la division du débit de gaz pur fourni par la source (10) aux trois lignes de gaz (7, 8, 9'), la sortie de cette troisième ligne étant raccordée, de même que la sortie de gaz de l'ensemble, à une ligne d'alimentation de l'analyseur.

11. Dispositif selon l'une quelconque des revendications 6 à 10, caractérisé en ce que les moyens (16) pour charger d'impuretés le courant de gaz pur dérivé circulant dans une des lignes dérivées sont constitués par une pluralité de cartouches de perméation ($16_i$) débitant sélectivement ou collectivement, en parallèle, des impuretés dans ladite ligne.

12. Dispositif selon l'une quelconque des revendications 6 à 11, caractérisé en ce qu'il comprend une ligne (4) de raccordement de l'analyseur à une source de gaz à analyser et un régulateur de débit (20) placé dans une ligne de décharge (24) de cette ligne (4).

13. Dispositif selon la revendication 12, caractérisé en ce qu'il comprend des moyens de commande de l'ensemble des régulateurs de débit montés dans des lignes de décharge, pour régler le taux de dilution des impuretés dans le gaz d'étalonnage et pour commuter sélectivement et séquentiellement l'alimentation de l'analyseur sur un courant de gaz à analyser, un courant de gaz d'étalonnage et un courant de gaz pur.

14. Dispositif selon l'une quelconque des revendications 8 à 10, caractérisé en ce que, entre la sortie du/des épurateur(s) (13, 15; 34) et l'entrée de l'analyseur (1), les lignes sont dépourvues de tout organe susceptible de polluer les courants de gaz circulant dans ces lignes.

15. Dispositif selon l'une quelconque des revendications 6 à 14, caractérisé en ce qu'il comprend un régulateur de pression (25) raccordé à une ligne de décharge (26) de la ligne d'alimentation (2) de l'analyseur (1) et un régulateur de débit (28) monté sur une ligne de sortie des gaz de l'analyseur (1).

16. Dispositif selon l'une quelconque des revendications 6 à 15, caractérisé en ce qu'il comprend des moyens pour assurer une circulation continue de gaz dans les diverses lignes d'alimentation de l'analyseur.

17. Dispositif selon l'une quelconque des revendications 6 à 16, caractérisé en ce que les restrictions calibrées sont des orifices calibrés.

**Patentansprüche**

1. Verfahren zur Zuführung eines Gases zu einem Analysegerät (1) für Spuren von Verunreinigungen in einem Gas, bei dem man dem Analysegerät nacheinander a) ein zu analysierendes Gas, b) ein Reingas und c) ein durch Verdünnen einer oder mehrerer Verunreinigungen in diesem Reingas erhaltenes Kalibriergas zuführt, dadurch gekennzeichnet, daß man dem Analysegerät das Kalibriergas zuführt, indem man:

(i) das Reingas in einen Satz von mindestens zwei parallel angeordneten Zweigleitungen (7, 8) einspeist, wobei die durch jede Zweigleitung strömende Durchflußmenge des abgezweigten Reingases dadurch erhalten wird, daß man die dem Satz zugeführte Reingasdurchflußmenge mit Hilfe von kalibrierten Drosseln (31, 32), die jeweils am Eingang jeder der Zweigleitungen angeordnet sind, aufteilt,
(ii) das abgezweigte Reingas aus einer (7) der Zweigleitungen mit einer vorbestimmten Menge mindestens einer Verunreinigung beaufschlagt, wodurch man nach Verdünnung mit dem abgezweigten Reingas aus der anderen Zweigleitung das Kalibriergas erhält, das über eine Speiseleitung dem Analysegerät zugeleitet wird,
(iii) die in den Satz eingespeiste Reingasdurchflußmenge mit Hilfe eines stromaufwärts (12) des Satzes angeordneten Durchflußreglers regelt.

2. Verfahren nach Anspruch 1, dadurch gekennzeichnet, daß man die am Gasausgang des Satzes erhaltene Kalibriergasdurchflußmenge mit einem in einer stromaufwärts des Gasausgangs des Satzes mit dem Ausgang der Zweigleitung für das mit Verunreinigungen beaufschlagte Gas verbundenen Austragsleitung (21) angeordneten Durchflußregler (17) und einem anderen, in einer mit dem Gasausgang des Satzes verbundenen Austragsleitung (22) angeordneten Durchflußregler (18) regelt.

3. Verfahren nach Anspruch 1 oder 2, dadurch gekennzeichnet, daß man das Kalibriergas am Ausgang des Satzes vor dem Erreichen des Analysegeräts durch Zufuhr einer zusätzlichen Menge Reingas (9, 9', 14, 15) weiter verdünnt.

4. Verfahren nach einem der Ansprüche 1 bis 3, dadurch gekennzeichnet, daß es sich bei den kalibrierten Drosseln um kalibrierte Öffnungen handelt.

5. Verfahren nach Anspruch 4, dadurch gekennzeichnet, daß man die kalibrierten Öffnungen im schallnahen Betriebsbereich verwendet.

6. Vorrichtung zur Durchführung des Verfahrens nach einem der Ansprüche 1 bis 5, enthaltend:

a) eine Reingasquelle (10);
b) einen Satz von mindestens zwei parallel angeordneten und von der Reingasquelle (10) gespeisten Gaszweigleitungen (7, 8), wobei der Ausgang jeder Zweigleitung an einen gemeinsamen Gasausgang angeschlossen ist, der den Gasausgang des Satzes darstellt, welcher mit einer Leitung (2) zur Speisung des Analysegeräts (1) verbunden ist;
c) Einrichtungen (16) zum Beaufschlagen des durch eine der Zweigleitungen strömenden abgezweigten Reingases mit einer vorbestimmten Menge mindestens einer Verunreinigung;
d) eine am Eingang jeder der Zweigleitungen angeordnete Drossel, die so kalibriert ist, daß die in den Satz eingespeiste Reingasdurchflußmenge in einem vorbestimmten Verhältnis zwischen den Zweigleitungen aufgeteilt wird;
e) einen Durchflußregler für in den Satz eingespeistes Reingas, der zwischen der Reingasquelle und dem Gaseingang des Satzes angeordnet ist,

wobei bei jeder Zweigleitung der Teil der Leitung zwischen dem Gaseingang des Satzes und der kalibrierten Drossel keinerlei Druckmeßeinrichtungen enthält.

7. Vorrichtung nach Anspruch 6, dadurch gekennzeichnet, daß sie außerdem auch noch zwei Durchflußregler (17, 18), die jeweils in einer stromaufwärts des Gasausgangs des Satzes mit dem Ausgang der Zweigleitung (7) für mit Verunreinigungen beaufschlagtes Gas bzw. mit dem Gasausgang des Satzes verbundenen Austragsleitung (21, 22) angeordnet sind, enthält.

8. Vorrichtung nach Anspruch 6 oder 7, dadurch gekennzeichnet, daß sie eine zwischen der Reingasquelle und dem Gaseingang des Satzes angeordnete Reinigungseinrichtung enthält.

9. Vorrichtung nach einem der Ansprüche 6 bis 8, dadurch gekennzeichnet, daß sie außerdem auch noch eine zusätzliche Leitung (9) enthält, die über einen Durchflußregler (14) und eine Reinigungseinrichtung (15) mit Reingas aus der Quelle (10) gespeist wird, wobei der Ausgang dieser Leitung (9) ebenso wie der Gasausgang des Satzes an die Leitung (2) zur Speisung des Analysegeräts (1) angeschlossen ist.

10. Vorrichtung nach Anspruch 6 oder 7, dadurch gekennzeichnet, daß sie außerdem auch noch eine dritte Gasleitung (9') enthält, wobei die drei Leitungen über den gleichen Durchflußregler (33) und die gleiche Reinigungseinrichtung (34) von der Reingasquelle (10) gespeist werden und der Eingang der dritten Leitung (9') mit einer kalibrierten Drossel (37) ausgestattet ist, die mit den beiden anderen (35, 36) die Aufteilung der den drei Gasleitungen (7, 8, 9') von der Quelle (10) zugeführten Reingasdurchflußmenge gewährleistet, wobei der Ausgang dieser dritten Leitung ebenso wie der Gasausgang des Satzes an eine Leitung zur Speisung des Analysegeräts angeschlossen ist.

11. Vorrichtung nach einem der Ansprüche 6 bis 10, dadurch gekennzeichnet, daß die Einrichtungen (16) zum Beaufschlagen des durch eine der Zweigleitungen strömenden abgezweigten Reingasstroms mit Verunreinigungen aus mehreren Permeationspatronen ($16_i$) bestehen, die selektiv oder gemeinsam in Parallelschaltung die Leitung mit den Verunreinigungen beschicken.

12. Vorrichtung nach einem der Ansprüche 6 bis 11, dadurch gekennzeichnet, daß sie eine Leitung (4) zum Anschluß einer Quelle von zu analysierendem Gas an das Analysegerät und einen in einer Austragsleitung (24) dieser Leitung (4) angeordneten Durchflußregler (20) enthält.

13. Vorrichtung nach Anspruch 12, dadurch gekennzeichnet, daß sie Steuereinrichtungen für den Satz von in den Austragsleitungen angebrachten Durchflußreglern zur Regelung der Verdünnung der Verunreinigungen im Kalibriergas und zur selektiven und sequentiellen Umschaltung der Speisung des Analysegeräts auf einen Strom von zu analysierendem Gas, einen Kalibriergasstrom und einen Reingasstrom enthält.

14. Vorrichtung nach einem der Ansprüche 8 bis 10, dadurch gekennzeichnet, daß die Leitungen zwischen dem Ausgang der Reinungseinrichtung(en) (13, 15; 34) und dem Eingang des Analysegeräts (1) keinerlei Einrichtungen enthalten, die die durch diese Leitungen strömenden Gasströme verunreinigen können.

15. Vorrichtung nach einem der Ansprüche 6 bis 14, dadurch gekennzeichnet, daß sie einen Druckregler (25), der an eine Austragsleitung (26) der Leitung (2) zur Speisung des Analysegeräts (1) angeschlossen ist, und einen Durchflußregler (28), der in einer Gasausgangsleitung des Analysegeräts (1) angebracht ist, enthält.

16. Vorrichtung nach einem der Ansprüche 6 bis 15, dadurch gekennzeichnet, daß sie Einrichtungen zur Gewährleistung einer kontinuierlichen Gasströmung durch die verschiedenen Leitungen zur Speisung des Analysegeräts enthält.

17. Vorrichtung nach einem der Ansprüche 6 bis 16, dadurch gekennzeichnet, daß es sich bei den kalibrierten Drosseln um kalibrierte Öffnungen handelt.

## Claims

1. Process for supplying gas to an analyser (1) of traces of impurities in a gas, according to which the analyser is supplied sequentially with a) a gas to be analysed, b) a pure gas and c) a standardizing gas obtained by dilution of one or more impurities in this pure gas, characterized in that the said standardizing gas is supplied to the analyser in the following manner:

(i) a set of at least two bypass lines (7, 8), arranged in parallel, is fed with the pure gas, the flow of bypass pure gas travelling in each bypass line being obtained by dividing the flow of pure gas which feeds the set with the aid of calibrated restrictions (31, 32) each of which is placed at the entry of each of the bypass lines,
(ii) the bypass pure gas, of one (7) of the bypass lines, is charged with a predetermined quantity of at least one impurity to obtain, after dilution with the bypass pure gas of the other bypass line, the standardizing gas, which is directed towards the analyser along a feed line,
(iii) the flow rate of pure gas feeding the set is regulated with the aid of a flow regulator placed upstream (12) of the set.

**2.** Process according to Claim 1, characterized in that the flow rate of standardizing gas obtained at the gas exit of the set is regulated with a flow regulator (17) placed in a discharge line (21) connected to the exit of the bypass line for gas charged with impurities upstream of the gas exit of the set and another flow regulator (18) placed in a discharge line (22) connected to the gas exit of the set.

**3.** Process according to Claim 1 or 2, characterized in that, before reaching the analyser, the standardizing gas is subjected, on leaving the set, to another dilution stage by the introduction of an additional quantity of pure gas (9, 9', 14, 15).

**4.** Process according to any one of Claims 1 to 3, characterized in that the said calibrated restrictions are calibrated orifices.

**5.** Process according to Claim 4, characterized in that the said calibrated orifices are employed in a sonic regime.

**6.** Device for making use of the process according to any one of Claims 1 to 5, including:

a) a source of pure gas (10);
b) a set of at least two bypass gas lines (7, 8) fitted in parallel and fed by the source (10) of pure gas, the exit of each bypass line being connected to a common gas exit constituting the gas exit of the set, which is connected to a feed line (2) of the analyser (1);
c) means (16) for charging the bypass pure gas travelling in one of the bypass lines, with a predetermined quantity of at least one impurity;
d) a restriction placed at the entry of each of the bypass lines, which is calibrated in order to divide in a predetermined ratio between the bypass lines the flow of pure gas feeding the set;
e) a flow regulator of pure gas feeding the set, situated between the source of pure gas and the gas entry of the set,

in the case of each bypass line the portion of line between the gas entry of the set and the calibrated restriction being devoid of any pressure-measuring member.

**7.** Device according to Claim 6, characterized in that it additionally includes two flow regulators (17, 18), each placed in a discharge line (21, 22) connected, in the case of one, to the exit of the bypass line (7) for gas charged with impurities, upstream of the gas exit of the set and, in the case of the other, to the gas exit of the set.

**8.** Device according to Claim 6 or 7, characterized in that it includes a purifier situated between the source of pure gas and the gas entry of the set.

**9.** Device according to any one of Claims 6 to 8, characterized in that it furthermore includes an additional line (9) fed with pure gas by the source (10) via a flow regulator (14) and a purifier (15), the exit of this line (9) being connected, just as the gas exit of the set, to the feed line (2) of the analyser (1).

**10.** Device according to Claim 6 or 7, characterized in that it additionally includes a third gas line (9'), the three lines being fed by the source (10) of pure gas via the same flow regulator (33) and the same purifier (34), the entry of the third line (9') being provided with a calibrated restriction (37) ensuring, with the other two (35, 36), the division of the flow of pure gas supplied by the source (10) to the three gas lines (7, 8, 9'), the exit of this third line being connected, just as the gas exit of the set, to a feed line of the analyser.

**11.** Device according to any one of Claims 6 to 10, characterized in that the means (16) for charging with impurities the stream of bypass pure gas travelling in one of the bypass lines consist of a plurality of permeation cartridges ($16_i$) dispensing impurities into the said line selectively or collectively, in parallel.

**12.** Device according to any one of Claims 6 to 11, characterized in that it includes a line (4) for connecting the analyser to a source of gas to be analysed and a flow regulator (20) placed in a discharge line (24) of this line (4).

**13.** Device according to Claim 12, characterized in that it includes means for control-driving the set of the flow regulators which are fitted in discharge lines, in order to regulate the dilution ratio of the impurities in the standardizing gas and in order to switch selectively and sequentially the feed of the analyser to a stream of gas to be analysed, a stream of standardizing gas and a stream of pure gas.

**14.** Device according to any one of Claims 8 to 10, characterized in that, between the exit of the purifier(s) (13, 15; 34) and the entry of the analyser (1) the lines are devoid of any member capable of contaminating the gas streams travelling in these lines.

**15.** Device according to any one of Claims 6 to 14, characterized in that it includes a pressure regulator (25) connected to a discharge line (26) of the feed line (2) of the analyser (1) and a flow regulator (28) fitted in an exit line for the gases from the analyser (1).

16. Device according to any one of Claims 6 to 15, characterized in that it includes means for ensuring a continuous travel of gas in the various feed lines of the analyser.

17. Device according to any one of Claims 6 to 16, characterized in that the calibrated restrictions are calibrated orifices.

FIG.:1

FIG.:2

FIG.:3